# EUROPEAN PATENT APPLICATION

(11) **EP 4 806 978 A1**
(43) Date of publication of application: **16.09.2026**
(21) Application number: 24917248.7
(22) Date of filing: 01.08.2024
(51) Int. Cl.: C10G 2/00, C10J 3/72, C01B 3/38, C01B 3/48, C01B 32/40, C07C 1/20, C07C 29/151, C07C 31/04

(54) **METHOD AND APPARATUS FOR PREPARING HYDROCARBONS**

(30) Priority: 12.01.2024 KR 20240005331
(71) Applicant: SK Innovation Co., Ltd., Seoul 03188 (KR)
(72) Inventor: JEON, Heejung, Daejeon 34124 (KR); KIM, Okyoun, Daejeon 34124 (KR); YUN, Dongmin, Daejeon 34124 (KR)
(74) Representative: Lorenz Seidler Gossel Part. mbB
(86) International application number: PCT/KR2024/095963
(87) International publication number: WO 2025/150759

(57) **Abstract**

The present disclosure provides a method for preparing hydrocarbons, comprising the steps of: (S1) heat treating organic waste so as to generate a first mixed gas; (S2) dry reforming the first mixed gas in a first fluidized bed reactor so as to generate a second mixed gas; (S3) separating the second mixed gas into a first stream that comprises carbon dioxide and a second stream that comprises hydrogen and carbon monoxide; (S4) introducing the first stream, which was separated out in step (S3), to a second fluidized bed reactor and converting same into carbon monoxide through a reverse Boudouard reaction; (S5) mixing the second stream and the carbon monoxide, which was converted in step (S4), so as to prepare a third mixed gas; (S6) generating syngas from the third mixed gas through a water-gas shift reaction; and (S7) preparing hydrocarbons from the syngas through a catalytic reaction.

## Description

### [Technical Field]

The present invention relates to a method and an apparatus for preparing a hydrocarbon from an organic waste, and more particularly, to a method and an apparatus for preparing a hydrocarbon, which may improve a manufacturing yield of a hydrocarbon from an organic waste and minimize carbon dioxide emissions.

### [Background Art]

Organic wastes may seriously damage the environment due to decomposition when landfilled and should be discarded through a prescribed treatment process after collecting them according to their properties when discarded. However, since simple disposal of the organic wastes requires securing treatment facilities and consuming a large amount of manpower and is more wasteful than productive, methods and technologies for recycling organic wastes have been developed in recent years. Representatively, a gasification process technology in which a syngas is produced using organic wastes and converted into a high value-added product for energization may be mentioned.

The gasification process generally refers to a series of processes of reacting carbonaceous raw materials such as coal, organic wastes, and biomass under the supply of water vapor, oxygen, carbon dioxide, or a mixture thereof to convert the raw materials into a syngas including hydrogen and carbon monoxide, in which the "syngas" refers to a mixed gas which is usually produced by a gasification reaction and includes hydrogen and carbon monoxide and may further include carbon dioxide and/or methane.

The gasification process technology has expanded to a technology of preparing fuels and raw materials of various compounds, and for example, the syngas may be used as the raw material of a Fischer-Tropsch synthesis reaction to manufacture high value-added products such as light oil, heavy oil, diesel oil, wax, jet oil, and lubricant base oil. Besides, it is known that the technology may be applied to hydrogen power generation, ammonia manufacture, an oil refining process, and the like, using a hydrogen in the syngas which is the main product of the gasification process, and high value-added chemicals such as acetic acid, olefin, dimethyl ether, aldehyde, fuel, and an additive may be obtained, using methanol manufactured from the syngas. However, since the syngas prepared from organic wastes has a very poor manufacturing yield, it is difficult to effectively prepare a high value-added compound material from the syngas.

Recently, as a process for manufacturing syngas, a gasification process using a catalyst has been carried out, but the catalyst is deactivated by the formation of coke and the like in the gasification process, and process trouble occurs due to the deactivated catalyst during a continuous operation. In addition, for securing economic feasibility, relatively expensive catalysts need to be recovered, but in order to recover catalyst discharged in the state in which coke and the like are agglomerated, a plurality of subsequent processes (such as air burning) should be carried out, and thus, process efficiency is significantly deteriorated.

In addition, since the conventionally performed gasification process of organic wastes has a significantly low syngas manufacturing yield of 30% or less, the syngas being converted into a high value-added product, and thus, has poor productivity, there are limitations to utilizing or commercializing the process. Further, in terms of environmental protection, it is preferred to suppress CO₂ emission, but since the gasification reaction product of organic wastes contains CO₂ in addition to H₂ and CO, more carbon dioxide is emitted as compared with the case of landfill or pyrolysis treatment, and thus, the gasification process has a serious problem of causing rather increased environmental pollution.

Thus, there is a need for a method and an apparatus for preparing a hydrocarbon, which may improve a manufacturing yield of syngas which may be converted into a high value-added product during a gasification process of organic wastes and efficiently convert the syngas into a high value-added hydrocarbon, while minimizing carbon dioxide emissions.

### [Disclosure]

### [Technical Problem]

An object of the present disclosure is to provide a method and an apparatus for preparing a hydrocarbon from an organic waste, which have a significantly improved manufacturing yield of hydrocarbon.

Another object of the present disclosure is to provide a method and an apparatus for preparing a hydrocarbon, which have a significantly improved process yield.

Still another object of the present disclosure is to provide a method and an apparatus for preparing a hydrocarbon, which may minimize carbon dioxide emissions.

### [Technical Solution]

In one general aspect, a method for preparing a hydrocarbon includes: (S1) heat treating an organic waste to produce a first mixed gas; (S2) dry reforming the first mixed gas in a first fluidized bed reactor to produce a second mixed gas; (S3) separating the second mixed gas into a first stream including carbon dioxide and a second stream including hydrogen and carbon monoxide; (S4) introducing the first stream separated in (S3) to a second fluidized bed reactor and converting the first stream into carbon monoxide through a reverse Boudouard reaction; (S5) mixing the second stream and the carbon monoxide converted in (S4) to prepare a third mixed gas; (S6) producing a syngas from the third mixed gas through a water gas shift reaction; and (S7) producing a hydrocarbon from the syngas through a catalytic reaction.

In an example, the first mixed gas may further include one or more selected from the group consisting of landfill gas, shale gas, refinery exhaust gas, and biogas.

In an example, the first stream may include 50 vol% or more of carbon dioxide.

In an example, the pyrolysis gas may have a C/O ratio of 0.1 or more.

In an example, (S2) may be performed under a composite catalyst in which an active metal is supported on a support.

In an example, the active metal may include one or more selected from the group consisting of nickel, vanadium, iron, platinum, palladium, and ruthenium.

In an example, the support may include one or more selected from the group consisting of silica, alumina, silica-alumina, carbon, zirconia, titania, zeolite, SAPO, and ALPO.

In an example, (S2) may be performed at a temperature of 700 to 1000°C.

In an example, (S4) may be performed at a temperature of 600 to 1000°C and a pressure of 50 to 300 KPa.

In an example, the syngas may include hydrogen and carbon monoxide, and a ratio between the hydrogen and the carbon monoxide may satisfy 1.8:1 to 2.2:1.

In an example, the organic waste in (S1) may be one or more selected from the group consisting of waste plastic, solid waste, biomass, waste oil, waste tire, and standard plastic garbage bags.

In an example, purifying the first mixed gas of (S1) may be further included before (S2).

In another general aspect, an apparatus for preparing a hydrocarbon includes: a pyrolysis reactor which heat treats an organic waste to produce a first mixed gas; a first fluidized bed reactor which dry reforms the first mixed gas under a catalyst to produce a second mixed gas; a carbon dioxide separation unit which separates the second mixed gas into a first stream including carbon dioxide and a second stream including hydrogen and carbon monoxide; a second fluidized bed reactor which converts the first stream into carbon monoxide through a reverse Boudouard reaction; a gas mixing unit which mixes the second stream and carbon monoxide converted in the second fluidized bed reactor to prepare a third mixed gas; a syngas production unit which converts the third mixed gas into a syngas through a water gas shift reaction; and a hydrocarbon conversion unit which converts the syngas into a hydrocarbon through a catalytic reaction.

In an example, the apparatus for preparing a hydrocarbon may further include a cyclone connected between the first fluidized bed reactor and the carbon dioxide separation unit; a catalyst supply line which connects the cyclone and the second fluidized bed reactor; and a catalyst recirculation line which connects the second fluidized bed reactor and the first fluidized bed reactor, wherein the cyclone separates the second mixed gas and the catalyst discharged from the fluidized bed reforming reactor, supplies the second mixed gas to the carbon dioxide separation unit, and supplies the catalyst to the second fluidized bed reactor.

In an example, a purification unit may be further included between the heat treatment reactor and the first fluidized bed reactor.

### [Advantageous Effects]

According to an exemplary embodiment of the present disclosure, a manufacturing yield of a hydrocarbon from organic wastes may be significantly improved.

According to an example of the present disclosure, hydrocarbon conversion may be performed using a process and an apparatus appropriate for a C/O element ratio of a feed, thereby significantly improving a manufacturing yield of hydrocarbon.

According to an exemplary embodiment of the present disclosure, carbon dioxide emissions may be minimized in a process of preparing a hydrocarbon.

### [Description of Drawings]

FIG. 1 is a schematic diagram showing an apparatus for preparing a hydrocarbon according to an example of the present disclosure.
FIG. 2 is a schematic diagram showing an apparatus for preparing a hydrocarbon including a purification process according to an example.
FIG. 3 is a schematic diagram showing an apparatus for preparing a hydrocarbon including a catalyst circulation process according to an example.

### [Best Mode]

The singular form of the term used herein may be intended to also include a plural form, unless otherwise indicated.

The numerical range used in the present specification includes all values within the range including the lower limit and the upper limit, all double limited values, and all possible combinations of the upper limits and the lower limits in the numerical range defined in different forms. Unless otherwise defined in the present specification, values which may be outside a numerical range due to experimental error or rounding off of a value are also included in the defined numerical range.

The term "comprise" mentioned in the present specification is an open-ended description having a meaning equivalent to the term such as "is/are provided", "contain", "have", or "is/are characterized", and does not exclude elements, materials, or processes which are not further listed.

The unit of % used in the present specification without particular mention refers to % by weight, unless otherwise defined.

As a conventional process for manufacturing syngas, a gasification process using a catalyst has been carried out, but due to the formation of coke and the like in the gasification process, the catalyst is deactivated causing process trouble by the deactivated catalyst during a continuous operation. In addition, for securing economic feasibility, relatively expensive catalysts need to be recovered, but in order to recover catalyst discharged in the state in which coke is agglomerated, a plurality of subsequent processes (such as air burning) should be carried out, which significantly deteriorates process efficiency, and a large amount of carbon dioxide is emitted during the process causing environmental pollution. In addition, since the conventionally performed gasification process of organic wastes has a significantly low manufacturing yield of syngas of 30% or less and poor productivity, there were limits to conversion of the syngas into a high value-added product. Thus, the inventors of the present disclosure devised a preparation method and a preparation apparatus which may efficiently prepare hydrocarbons from organic wastes while minimizing carbon dioxide emissions.

The present disclosure provides a method for preparing hydrocarbons, comprising the steps of: (S1) heat treating organic waste so as to generate a first mixed gas; (S2) dry reforming the first mixed gas in a first fluidized bed reactor so as to generate a second mixed gas; (S3) separating the second mixed gas into a first stream that comprises carbon dioxide and a second stream that comprises hydrogen and carbon monoxide; (S4) introducing the first stream, which was separated out in (S3), to a second fluidized bed reactor and converting same into carbon monoxide through a reverse Boudouard reaction; (S5) mixing the second stream and the carbon monoxide, which was converted in (S4), so as to prepare a third mixed gas; (S6) generating syngas from the third mixed gas through a water-gas shift reaction; and (S7) preparing hydrocarbons from the syngas through a catalytic reaction.

Since the method for preparing a hydrocarbon according to the present disclosure may convert organic waste plastics into a syngas with high efficiency as compared with a conventional gasification process, a yield of a high value-added hydrocarbon converted from the syngas may be maximized. In addition, since carbon dioxide occurring in a process of preparing a syngas may be converted into a raw material of a syngas, carbon dioxide emission may be minimized in a hydrocarbon preparation process to prevent environmental pollution.

(S1) is a step of heat treating organic wastes to produce a first mixed gas, in which a gasification reaction of the organic wastes may occur.

In an example, the organic waste in (S1) may be one or more selected from the group consisting of waste plastic, solid waste, biomass, waste oil, waste tire, and standard plastic garbage bags.

Specifically, in (S1), any one or more gasification reactions selected from the following Reaction Formulae 1 to 4 may be involved:

[Reaction Formula 1] CₓH_{y} + H₂O → H₂ + CO (water gasification reaction)

[Reaction Formula 2] CₓH_{y} + CO₂ → CO (carbon dioxide gasification reaction)

[Reaction Formula 3] CO + 3H₂ → CH₄ + H2O (methanation reaction)

[Reaction Formula 4] CₓH_{y} + O₂→ CO₂ (oxidation reaction) .

In an example, the first mixed gas may include methane, hydrogen, carbon monoxide, and carbon dioxide, and may include various impurities such as nitrogen oxides, sulfur oxides, and hydrogen chloride.

In an example, (S1) may be performed in a first catalyst condition or a non-catalytic condition in order to increase a methane content in the composition of the first mixed gas. Since it may be advantageous in terms of methane reforming reaction efficiency or a syngas manufacturing yield to increase a methane content in the composition of the first mixed gas, in order to increase the methane content in (S1), an acid site catalyst or molybdenum series forming catalyst may be used as a bed material of a gasifier. When the catalyst is not used, the operation may be performed with an increased methane content by operating the gasifier under the low temperature and high pressure conditions. The yield improvement effect from methane reforming may be expected identically in a C2 to C4 hydrocarbon gas content as well as the methane content, and the C2 to C4 gas content may also be increased by using the acid site catalyst or through low temperature and high pressure gasification operation.

In an example, the first catalyst may be an acid site catalyst or molybdenum-based forming catalyst. The acid site catalyst may be alumina or a catalyst having a solid acid site derived from a structure. The alumina may be alumina alone, silica-alumina, alumina dispersed in a carbon structure, and the like, and the structure acid site material may be zeolite, SAPO, AlPO, MOF, a structural variant thereof, and the like. The molybdenum-based catalyst refers to a catalyst in which molybdenum is supported on a support, and if necessary, nickel, cobalt, and the like may be added, or tungsten may be used instead of molybdenum. Any support may be used as long as it has durability to support an active metal, and for example, it may include materials including one or more selected from silica, alumina, silica-alumina, carbon, and zirconia.

As another example of increasing the methane content in the first mixed gas, the first mixed gas may further include one or more selected from the group consisting of landfill gas, shale gas, refinery exhaust gas, and biogas. Since the landfill gas, shale gas, refinery exhaust gas, and biogas include 40 vol% or more, specifically 50 vol% or more of methane and carbon dioxide, the first mixed gas further includes the gas described above, and thus, the manufacturing yield of syngas may be further improved through a methane reforming reaction and a reverse Boudouard reaction which are subsequent processes.

In an example, a C/O element ratio of the first mixed gas may be 0.8 or less, 0.75 or less, or 0.7 or less as the upper limit and 0.1 or more, 0.15 or more, or 0.2 or more as the lower limit. Specifically, it may be 0.1 to 0.8, more specifically 0.2 to 0.7.

The method for preparing a hydrocarbon according to the present disclosure may perform a reforming reaction of methane well, even when the first mixed gas includes a relatively high C/O element ratio as in the range described above. Specifically, since the catalyst used in the methane reforming reaction forms the circulation process described later, an effect of continuously performing the reaction regardless of the deactivation of the catalyst by coke which may be produced by dry reforming is exhibited.

In an example, a step of purifying the first mixed gas may be further included, after (S1).

The first mixed gas produced by heat treating organic wastes may include one or two or more impurities selected from the group consisting of tar, sulfur, nitrogen, and chlorine. Specifically, the first mixed gas may include water-soluble impurities such as H₂S, HCl, HOCl, and NH₃ and water-insoluble impurities such as tar. Since these impurities included in the first mixed gas may induce deactivation of the catalyst to reduce the efficiency of a subsequent process, when purification is performed after removing impurities from the first mixed gas, the efficiency of the entire process may be improved.

A method for purifying the first mixed gas may be one or a combination of two or more selected from the group consisting of use of a dust collection filter for high pressure, water washing, basic solution washing, passing through a ceramic filter, and the like, but is not limited thereto. When the water washing or basic solution washing is used, water-soluble impurities such as H₂S, HCl, HOCl, and NH₃ included in the first mixed may be removed, and when the first mixed gas is passed through a ceramic filter or a dust collection filter, water-insoluble impurities such as tar, dust, and the like may be removed.

(S2) is a step of dry reforming methane contained in the first mixed gas in a first fluidized bed reactor to prepare a second mixed gas. In (S2), a reforming reaction according to the following Reaction Formula 5 may be involved:

[Reaction Formula 5] CH₄ + CO₂ → 2CO + 2H₂ (carbon dioxide reforming reaction).

The reforming reaction of (S2) may be performed at a temperature of 600°C to 1400°C and a pressure of 30 KPa to 2000 KPa.

(S2) may be operated without a catalyst, but may be operated using a catalyst in order to increase a reaction conversion rate at a low temperature of 600°C to 700°C. The catalyst of (S2) may be a composite catalyst in which an active metal is supported on a support. The active metal may include one or more selected from the group consisting of nickel, vanadium, iron, platinum, palladium, and ruthenium. Usually, the active metal may be nickel, vanadium, or iron, and when an impurity content in a raw material such as organic wastes is low in the heat treatment process, precious metals such as platinum, palladium, or ruthenium may be used.

In an example, the support may be a solid acid material such as an oxide or zeolite, and specifically, may be one or more selected from the group consisting of ZSM-5, ZSM-11, USY zeolite, ferrierite, mordenite, MCM-22, SUZ-4, L-type zeolite, silica, alumina, silica-alumina, carbon, zirconia, and titania.

Since the dry reforming reaction is performed by the methane reforming reaction of (S2), the reforming efficiency of methane is improved, thereby increasing the yield of the hydrocarbon. Specifically, a ratio of hydrogen : carbon monoxide of the dry reforming process is close to 1:1, the catalytic reaction of the syngas as a subsequent process may be performed better.

In addition, since (S2) is performed in the fluidized bed reactor, the catalyst used in the methane reforming reaction may be added to a reverse Boudouard reaction process as a subsequent process and form a circulation process of being resupplied after regeneration. Since the methane reforming reaction is performed in a dry manner in (S2), coke is accumulated in the catalyst to deactivate a catalytic reaction, but (S2) is performed in the fluidized bed reactor to form a circulation process of regenerating and resupplying a catalyst, and thus, a continuous reforming reaction is allowed in spite of methane dry reforming.

(S3) is a step of separating the second mixed gas into a first stream including carbon dioxide and a second stream including hydrogen and carbon monoxide.

In an example, a method for separating the second mixed gas into a first stream and a second stream is not limited as long as it is known in the art, but in the present disclosure, the separation may be performed using a carbon dioxide separation unit, and the carbon dioxide separation unit may be an amine scrubber. Usually, the amine scrubber binds and removes carbon dioxide through an amine-based material, and since it may separate components such as carbon dioxide and hydrogen sulfide from gas steam and recover gas containing hydrogen, carbon monoxide, or inert gas, when the amine scrubber is used, the first mixed gas may be separated into the first stream and the second stream.

As another example, the carbon dioxide separation unit may be a carbon capture and storage (CCS) unit. When the CCS unit is used for separation of carbon dioxide, it may adsorb and separate carbon dioxide using an adsorbent including any one or two or more selected from calcium oxide, calcium hydroxide, dolomite, limestone, or trona, and thus, the second mixed gas may be separated into the first and second streams using the carbon capture and storage (CCS) unit.

The first stream may include carbon dioxide at 40 vol% or more, 50 vol% or more, or 60 vol% or more as the lower limit and 99 vol% or less, 90 vol% or less, 80 vol% or less, or 70 vol% or less as the upper limit. Specifically, the first stream may include 40 to 99 vol%, more specifically 50 to 80 vol% of carbon dioxide. In the carbon dioxide separation unit, when carbon dioxide is captured and then separated, in order to separate carbon dioxide to a high purity, a regeneration tower in which carbon dioxide separation from an adsorbing material occurs should be designed with a high stage, and thus, more energy is consumed. Therefore, since the first stream includes carbon dioxide in the range described above, a carbon dioxide separation process may be performed under slightly milder conditions.

(S4) is a step of converting the first stream separated in (S3) into carbon monoxide through a reverse Boudouard reaction in a second fluidized bed reactor. Since carbon dioxide contained in the first stream is further converted into carbon monoxide through the reverse Boudouard reaction, environmental pollution may be prevented in terms of decreasing emitted carbon dioxide, while maximizing the yield of syngas. The reverse Boudouard reaction may involve the following Reaction Formula 6:

[Reaction Formula 6] C+CO₂ → 2CO.

(S4) may be performed at a temperature of 600°C to 1000°C and a pressure of 50 KPa to 300 KPa.

In an example, a carbon source in the reverse Boudouard reaction in (S4) may be a catalyst in which coke supplied in (S2) is deposited, and if necessary, the reaction may be performed by supplying a carbon source such as activated carbon from the outside.

Specifically, the catalyst in which coke used in the methane dry reforming reaction in (S2) is deposited may be used in the reverse Boudouard reaction of (S4). The coke deposited in the catalyst may be used as a carbon source of the reverse Boudouard reaction to regenerate the catalyst. The regenerated catalyst may be used in the methane dry reforming reaction of (S2) again. When the catalyst circulation process is used, the methane dry reforming reaction may be continuously performed, the carbon source required in the reverse Boudouard reaction does not need to be supplied from the outside, and thus, an economical process may be performed.

That is, since the first fluidized bed reactor and the second fluidized bed reactor in which a methane reforming reaction and a reverse Boudouard reaction are performed form circulation process while the yield of the syngas may be maximized through the methane reforming reaction and the reverse Boudouard reaction in the present disclosure, a both continuous and economical process operation according to the catalyst regeneration may be implemented.

In an example, the external carbon source may be char derived from the organic wastes, and more specifically, may be char derived from pyrolysis of waste plastic or biomass-derived char. Otherwise, in order to prevent a possibility of impurity gases flowing in by an external carbon source, high-purity graphite may be included.

(S5) is a step of mixing the second stream separated in the carbon dioxide separation unit and carbon monoxide converted by the reverse Boudouard reaction in (S4) to produce a third mixed gas.

The third mixed gas may include hydrogen and carbon monoxide.

(S6) is a process of adjusting a ratio between carbon monoxide and hydrogen in the third mixed gas through a water gas shift reaction to produce a syngas. The third mixed gas may be converted through the water gas shift reaction so as to satisfy a ratio of hydrogen : carbon monoxide appropriate for a catalytic reaction as a subsequent process. The water gas shift reaction may involve the following Chemical Formula 7:

[Chemical Formula 7] CO + H₂O → H₂ + CO₂.

The water gas shift reaction may be performed under a catalyst including Fe and Cr. The water gas shift reaction may be under pressure performed at a temperature of 100°C to 400°C, specifically 100°C to 300°C and at 20 bar to 80 bar, specifically 25 bar to 70 bar.

The syngas produced by the water gas shift reaction may have a ratio of hydrogen : carbon monoxide of 1.5 to 3:1, specifically 1.9 to 2.1:1. Since the ratio of hydrogen : carbon monoxide in the syngas satisfies the range described above, a catalytic reaction process which is a subsequent process may be performed well.

(S7) is a step of converting the syngas produced in (S6) into a hydrocarbon oil fraction, in which the syngas may be converted into an appropriate hydrocarbon oil fraction by a catalytic reaction.

The catalytic reaction is not limited as long as it may convert the syngas into a hydrocarbon oil fraction, but specifically, may be a Fischer-Tropsch reaction or a methanol and olefin conversion reaction.

In an example, when the catalytic reaction of (S7) is the Fischer-Tropsch reaction, a reaction involving the following Chemical Formula 8 may be performed using the syngas produced in (S6) as a raw material:

[Chemical Formula 8] nCO + 2nH₂ → CₙH₂ₙ + nH₂O.

The Fischer-Tropsch reaction may be performed under a catalyst including cobalt, nickel, or iron, may include alumina, silica, titania, or the like as a support, and may include a precious metal such as Pt, Ru, and Re as a cocatalyst.

The Fischer-Tropsch reaction may be performed at a temperature of 100°C to 500°C, specifically 200°C to 350°C, and at a pressure of 10 atm to 50 atm or 10 atm to 30 atm.

In an example, when the catalytic reaction of (S7) is a methanol and olefin conversion reaction, (S7) may include a reaction of converting the syngas into methanol; and converting methanol into olefin.

The reaction of converting the syngas into methanol may involve the following Chemical Formula 9:

[Chemical Formula 9] CO + 2H₂ → CH₃OH.

In an example, the reaction of converting the syngas into methanol may be performed under a Cu-based catalyst. Specifically, the Cu-based catalyst may be a Cu-based methanol-based synthetic catalyst, and as a support of the Cu-based catalyst, one or more selected from the group consisting of SiO₂, ZrO₂, Ga₂O₃ Al₂O₃, MgO, and TiO₂ may be used. Specifically, the Cu-based methanol-based synthetic catalyst may be Cu/Zn/Al₂O₃.

The reaction of converting the syngas into methanol may be performed at 400°C to 600°C, specifically 430°C to 530°C, and at 0.1 MPa to 10 MPa, specifically 0.1 MPa to 5 MPa.

The reaction of converting methanol into olefin may be performed under a zeolite-based catalyst or an AlPO₄-based molecular sieve catalyst. Specifically, the zeolite-based catalyst may be ZSM-5, and the AlPO₄-based catalyst may be a silicoaluminaphosphate (SAPO) molecular sieve catalyst, specifically, one or more selected from SAPO-5, SAPO-8, SAPO-11, SAPO-16, SAPO-17, SAPO-18, SAPO-20, SAPO-31, SAPO-34, SAPO-35, SAPO-44, and SAPO-46.

The reaction of converting methanol into olefin may be performed at a temperature of 200°C to 600°C, specifically 300°C to 500°C and at a pressure of 1 bar to 10 bar, specifically 1 bar to 5 bar.

The present disclosure provides an apparatus for preparing a hydrocarbon including: a pyrolysis reactor 10 which heat treats an organic waste 100 to produce a first mixed gas 110; a first fluidized bed reactor 20 which dry reforms the first mixed gas 110 under a catalyst to produce a second mixed gas 200; a carbon dioxide separation unit 30 which separates the second mixed gas 200 into a first stream 210 including carbon dioxide and a second stream 211 including hydrogen and carbon monoxide; a second fluidized bed reactor 40 which converts the first stream 210 into carbon monoxide through a reverse Boudouard reaction; a gas mixing unit 50 which mixes the second stream 211 and carbon monoxide converted in the second fluidized bed reactor 40 to prepare a third mixed gas 220; a syngas production unit 60 which converts the third mixed gas 220 into a syngas 230 through a water gas shift reaction; and a hydrocarbon conversion unit 70 which converts the syngas 230 into a hydrocarbon through a catalytic reaction.

The apparatus for preparing a hydrocarbon 1 according to the present disclosure may prepare syngas and hydrocarbons efficiently and economically, using a fluidized bed reactor when the methane reforming reaction and the reverse Boudouard reaction are performed, and may significantly decrease carbon dioxide emissions.

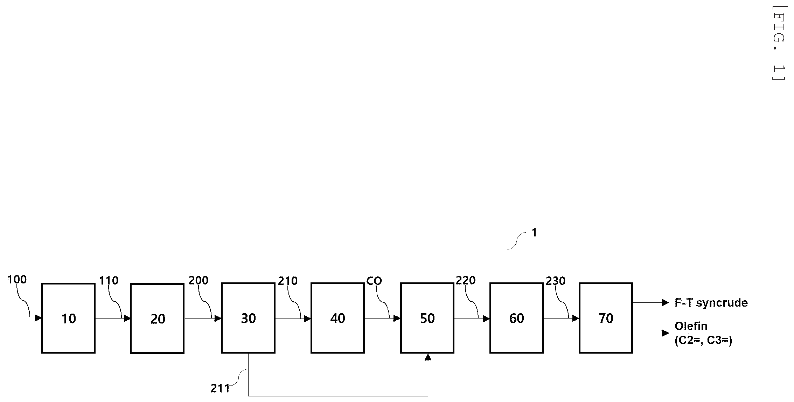

Referring to FIG. 1, the organic wastes 100 are introduced into a pyrolysis reactor 10 and heat treated to produce the first mixed gas 110. The first mixed gas 110 is introduced to the first fluidized bed reactor 20, and is converted into the second mixed gas 220 by the dry reforming reaction.

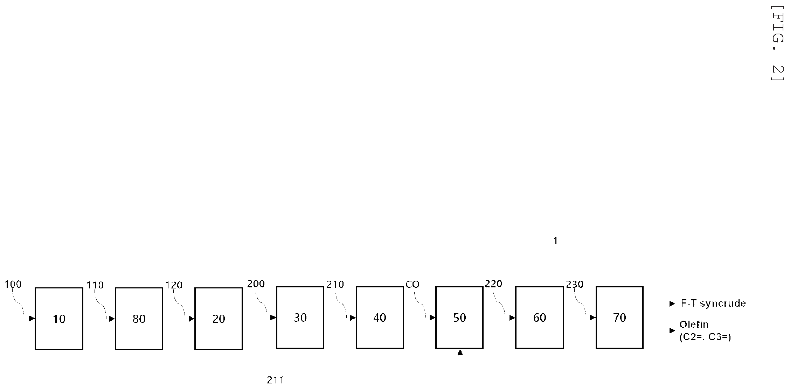

In an example, the hydrocarbon apparatus may further include a purification unit 80 connected between the pyrolysis reactor 10 and the first fluidized bed reactor 20. Specifically, referring to FIG. 2, the first mixed gas 110 flows in the purification unit 80 to remove impurities, and the first mixed gas 120 with impurities removed is introduced to the first fluidized bed reactor 20 to perform a methane reforming reaction.

The purification unit 80 may remove water-soluble impurities such as H₂S, HCl, HOCl, and NH₃ by being supplied with the first mixed gas 110 from the pyrolysis reactor 10 by including a sprayer which sprays a liquid and bringing the first mixed gas 110 and a weakly basic solution including water or sodium carbonate into contact, may remove dust using a dust collection filter for high pressure, or may remove water-insoluble impurities such as tar by including a ceramic filter.

The second mixed gas 200 flows in a carbon dioxide separation unit 30 and is separated into the first stream 210 including carbon dioxide and the second stream 211 including hydrogen and carbon monoxide. The second stream 211 is supplied directly to a gas mixing unit 50, and the first stream 210 is supplied to the second fluidized bed reactor 40 and converted into carbon monoxide through the reverse Boudouard reaction.

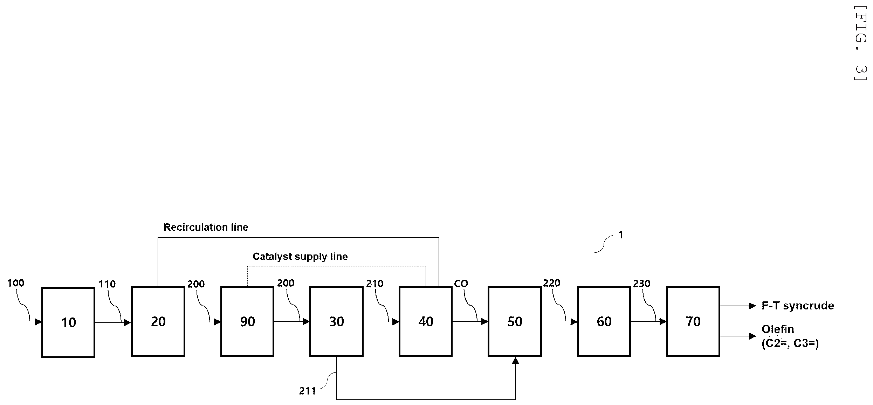

In an example, Referring to FIG. 3, a cyclone 90 connected between the first fluidized bed reactor 20 and the carbon dioxide separation unit 30; a catalyst supply line connecting the cyclone 90 and the second fluidized bed reactor 40; and a catalyst recirculation line connecting the second fluidized bed reactor 40 and the first fluidized bed reactor 20 are further included, in which the cyclone 90 may separate the second mixed gas 200 discharged from the first fluidized bed reactor 20 and the catalyst, supply the second mixed gas 200 to the carbon dioxide separation unit 30, and supply the catalyst to the second fluidized bed reactor 40.

Since the first fluidized bed reactor 20 and the second fluidized bed reactor 40 form a circulation process, a continuous process operation due to catalyst regeneration is allowed. In addition, since the catalyst in which coke used in the first fluidized bed reactor 20 is deposited may be used without separately supplying a carbon source for performing the reverse Boudouard reaction from the outside to the second fluidized bed reactor 40, a more economical process operation is allowed.

The gas mixing unit 50 mixes the second stream 211 and carbon monoxide converted in the second fluidized bed reactor 40 to prepare a third mixed gas 220. The third mixed gas 220 is supplied to the water gas conversion unit 60 and converted into a syngas 230 by adjusting a ratio of hydrogen : carbon monoxide in the third mixed gas 220 through a water gas shift reaction.

The syngas 230 may be introduced to the hydrocarbon conversion unit 70, converted into a hydrocarbon through a Fischer-Tropsch reaction or a methanol and olefin conversion reaction, and recovered as a high value-added oil fraction.

In an example, when the hydrocarbon conversion unit 70 performs a methanol and olefin conversion reaction, the hydrocarbon conversion unit 70 may include a methanol conversion unit which is supplied with the syngas 230 and converts the syngas into methanol and an olefin conversion unit which is supplied with methanol from the methanol conversion unit and converts methanol into an olefin.

In an example, the apparatus for preparing a hydrocarbon may be connected to a hydrocarbon conversion unit 70 and further include a separation step of separating the produced hydrocarbon into fractions at each boiling point through distillation.

Hereinafter, the specific examples of the present disclosure will be further described.

### (Example 1)

1000 g of municipal solid wastes were added to a pyrolysis reactor, and water vapor was introduced at a temperature of 1200°C and a pressure of 250 kPa under an alumina bed and then heat treated to recover a first mixed gas having a C/O element ratio of 0.7. The composition of the recovered first mixed gas is shown in the following Table 1, and the composition of a trace amount of impurity gas in the first mixed gas is shown in the following Table 2.

**[Table 1]**

| Feed | mol% | wt% |
|---|---|---|
| H₂ | 32 | 3 |
| CO | 16 | 18 |
| CO₂ | 40 | 72 |
| CH₄ | 11 | 7 |
| Total | 100 | 100 |

**[Table 2]**

| Impurity Gas | ppm |
|---|---|
| HCl | 2231 |
| H₂S | 1217 |
| COS | 231 |
| NH₃ | 3701 |

The first mixed gas was cooled, added to a purification unit (wet scrubber), and treated at 70°C and 100 KPa to remove impurities, and a purified first mixed gas was recovered. As a result of treating the first mixed gas including impurities with the composition of Table 2 through a wet scrubber, impurity gas such as NH₃, HCl, H₂S, and COS was not analyzed, and thus, it was confirmed that the impurities had been effectively removed.

The first mixed gas from which impurities had been removed was supplied to a first fluidized bed reactor provided with a Ni/Al₂O₃ catalyst at a space velocity of 2 L/g_{cat} ·h, thereby preparing a second mixed gas through methane dry reforming.

The Ni/Al₂O₃ fluidized bed catalyst was prepared by the following procedures. 1 part by weight of formic acid was added to a mixed solution of 10 parts by weight of pseudo-boehmite alumina and 95 parts by weight of nickel nitrate hexahydrate based on 100 parts by weight of water with stirring, and the reaction was performed for 3 hours to perform gelation, thereby preparing a precursor gel. 30 parts by weight of clay and 1.5 parts by weight of a MgO oxide were added and mixed with a homogenizer to prepare a solid mixture, which was mixed with the precursor gel, and then 10 parts by weight of colloidal silica (Ludox AS40, Aldrich) was added, 5 parts by weight of water was further added, and vigorous stirring was performed to prepare a composite catalyst sol. The composite catalyst sol was spray dried to prepare a fluidized bed catalyst.

The first fluidized bed reactor was filled with 100 g of the Ni/Al₂O₃ catalyst, reduction was performed for 2 hours while H₂ was supplied at a flow rate of 3.03 Nl/min at 900°C, and then operation was performed at a throughput of 2.0 L/g_{cat} ·h while the first mixed gas purified to a total flow rate of 3.33 Nl/min was introduced to recover the second mixed gas.

The catalyst was separated from the second mixed gas of the first fluidized bed reactor using a cyclone, CO₂ was separated using an amine scrubber, and a second stream from which the catalyst and carbon dioxide had been separated was recovered. Specifically, the second mixed gas was introduced to the first amine scrubber to capture CO₂ in an aqueous solution (amine solution) in which monoethanolamine (MEA) was dissolved at 50°C, and uncaptured gas was recovered as a second stream. The amine solution in the first amine scrubber was introduced to the second amine scrubber and separated into an amine solution and CO₂ at 100°C, and CO₂ was recovered as a first stream. The recovered first stream was introduced to the second fluidized bed reactor and converted into carbon monoxide through the reverse Boudouard reaction. The reverse Boudouard reaction was used in the dry reforming process and performed in the fluidized bed reactor filled with a Ni/Al₂O₃ catalyst in which active carbon and coke were deposited, by supplying the first stream at a space velocity of 2 L/g_{cat} ·h. At this time, the catalyst activated by removing coke through the reverse Boudouard reaction was added again to the first fluidized bed reactor through a recirculation line. An insufficient carbon source was replenished by further adding high-purity graphite. Unconverted CO₂ after the reverse Boudouard reaction was separately recovered by the amine scrubber.

The carbon monoxide converted from the first stream and the second stream were introduced to a mixing unit and were mixed at 200°C to produce a third mixed gas.

The third mixed gas was introduced to a syngas production unit to prepare a syngas having a mole ratio of H₂:CO=2:1 through a water gas shift reaction.

The syngas was supplied to a hydrocarbon conversion unit, and a hydrocarbon fraction was recovered by setting an injection speed so that the space velocity was 5000L/kg_{cat}·h and a volume of carbon monoxide : hydrogen : argon was 63.2:31.3:5.5 and performing a Fischer-Tropsch reaction at a reaction temperature of 300°C and a pressure of 10 bar for 60 hours, under a Co/ZnO (cobalt zinc oxide)-based catalyst.

### (Comparative Example 1)

A hydrocarbon was prepared and then recovered in the same manner as in Example 1, except that the reverse Boudouard reaction was not performed.

### (Comparative Example 2)

The process was performed in the same manner as in Example 1, except that the water gas shift reaction was not performed.

### (Experimental Example 1) Analysis of gas composition

When the hydrocarbon was prepared by the methods of Example 1, Comparative Example 1, and Comparative Example 2, the compositions of the gases included in the first mixed gas, the second mixed gas, the third mixed gas, and the syngas were analyzed and are shown in the following Table 3, and the composition of the main components included in the hydrocarbon prepared therefrom was analyzed and is shown in Table 4. Gas composition analysis was performed through gas chromatography (GC), and the total amount of gas was confirmed through a gas meter. Specifically, quantification was performed by GC to calculate selectivity by gas, and each composition by gas was analyzed by the total amount of gas confirmed by the gas meter.

**[Table 3]**

| | Composition | Example 1 | Comparative Example 1 | Comparative Example 2 |
|---|---|---|---|---|
| First mixed gas (mol) | H₂ | 6.91 | 6.91 | 6.91 |
| | CO | 3.46 | 3.46 | 3.46 |
| | CH₄ | 2.39 | 2.39 | 2.39 |
| | CO₂ | 8.92 | 8.92 | 8.92 |
| | H₂O | 0 | 0 | 0 |
| Second mixed gas (mol) | H₂ | 9.42 | 9.42 | 9.42 |
| | CO | 9.58 | 9.58 | 9.58 |
| | CH₄ | 0.13 | 0.13 | 0.13 |
| | CO₂ | 4.42 | 4.42 | 4.42 |
| | H₂O | 1.93 | 1.93 | 1.93 |
| Second stream (mol) | H₂ | 9.34 | - | 9.34 |
| | CO | 9.48 | - | 9.48 |
| | CH₄ | 0.1 | - | 0.1 |
| | CO₂ | 0.01 | - | 0.01 |
| Third mixed gas (mol) | H₂ | 9.31 | - | 9.31 |
| | CO | 18.03 | - | 18.03 |
| | CH₄ | 0.1 | - | 0.1 |
| | CO₂ | 0.09 | - | 0.09 |
| Syngas (mol) | H₂ | 18.11 | 11.38 | - |
| | CO | 8.81 | 5.71 | - |
| | CH₄ | 0.01 | 0.01 | - |
| | CO₂ | 0.07 | 0.15 | - |

**[Table 4]**

| | Example 1 | Comparative Example 1 | Comparative Example 2 |
|---|---|---|---|
| Gas (g) | 101.6 | 65.8 | 54.8 |
| Liquid, Wax (g) | 76.8 | 48.6 | 39.7 |
| H₂O (g) | 99.2 | 60.8 | 47.8 |

As shown in Tables 3 and 4, when a hydrocarbon was prepared by the method of Example 1, carbon dioxide included in the second mixed gas is recovered through the reverse Boudouard reaction and converted into carbon monoxide, and thus, the production amount of the syngas and the hydrocarbon prepared therefrom was increased, and high-quality syngas may be obtained. In particular, a circulation process in which a fluidized bed reactor is adopted as a reactor of a methane reforming and reverse Boudouard process to use a catalyst which has been deactivated by a carbon component deposited during the methane reforming reaction as the catalyst of the reverse Boudouard process, and the regenerated catalyst through the reverse Boudouard process is added again to the methane reforming reaction is allowed.

In addition, the syngas of Example 1 prepared by performing the dry reforming reaction, the reverse Boudouard reaction, and the water gas conversion process sequentially was measured to have high contents of H₂ and CO than that of CH₄ and CO₂. In addition, since the syngas manufacturing yield was improved, a liquid yield of the hydrocarbon prepared using the syngas was as high as 76.8 g. Thus, high value-added syngas and hydrocarbon may be prepared from a mixed gas formed by pyrolysis of organic wastes with a high yield from the method for preparing a hydrocarbon of the present disclosure, and also, since greenhouse gas emissions are reduced, an excellent environmental pollution prevention effect is shown.

However, in Comparative Example 1, since carbon dioxide included in the second mixed gas was not recovered through the reverse Boudouard reaction, the amount of the syngas obtained was small compared with Example 1, and since a large amount of carbon dioxide was included in the syngas, a hydrocarbon oil liquid yield was as low as 48.6g. In Comparative Example 2, since a water gas conversion process of the third mixed gas was not performed, a mole ratio of hydrogen : carbon monoxide of the syngas was not able to be controlled, and thus, since the hydrocarbon conversion reaction through the Fischer-Tropsch reaction was not performed well, the yield of the hydrocarbon liquid was measured as low as 39.7g.

Therefore, when the hydrocarbon was prepared by the method according to the present disclosure, process efficiency was improved, and thus, the syngas and the hydrocarbon were able to be prepared in a high yield. Specifically, since the methane dry reforming and the reverse Boudouard reaction process are performed using the fluidized bed reactor, the catalyst which has performed the methane reforming reaction may be added to the reverse Boudouard reaction process as a subsequent process and regenerated. Thus, since the circulation process of regenerating and resupplying the catalyst even when the catalyst is deactivated by coke in the dry reforming process may be performed, a continuous reaction is allowed even in the dry reforming. In particular, since the reverse Boudouard reaction separates carbon dioxide included in the mixed gas and converts carbon dioxide into carbon monoxide, carbon dioxide emissions may be reduced, and also a large amount of carbon monoxide may be prepared from carbon dioxide. In addition, since the syngas having a controlled ratio of hydrogen and carbon monoxide included in the mixed gas is prepared through the water gas conversion process after the reverse Boudouard reaction process, the catalytic reaction as a subsequent process is efficiently performed, and thus, the production yield of the syngas and hydrocarbon may be significantly improved.

Hereinabove, although the present disclosure has been described by the specific matters and limited exemplary embodiments in the present disclosure, they have been provided only for assisting the entire understanding of the present disclosure, and the present disclosure is not limited to the exemplary embodiments, and various modifications and changes may be made by those skilled in the art to which the present disclosure pertains from the description.

Therefore, the spirit of the present disclosure should not be limited to the above-described exemplary embodiments, and the following claims as well as all modifications equal or equivalent to the claims are intended to fall within the scope and spirit of the disclosure.

### [Detailed Description of Main Elements]

| | | | |
|---|---|---|---|
| 1: | Apparatus for preparing a hydrocarbon | 10: | Pyrolysis reactor |
| 20: | First fluidized bed reactor | 30: | Carbon dioxide separation unit |
| 40: | Second fluidized bed reactor | 50: | Gas mixing unit |
| 60: | Syngas production unit | 70: | Hydrocarbon conversion unit |
| 80: | Purification unit | 90: | Cyclone |
| 100: | Organic waste | 110: | First mixed gas |
| 120: | First mixed gas with impurity removed | 200: | Second mixed gas |
| 210: | First stream | 211: | Second stream |
| 220: | Third mixed gas | 230 | Syngas |

## Claims

1. A method for preparing a hydrocarbon, the method comprising:
(S1) heat treating an organic waste to produce a first mixed gas;
(S2) dry reforming the first mixed gas in a first fluidized bed reactor to produce a second mixed gas;
(S3) separating the second mixed gas into a first stream including carbon dioxide and a second stream including hydrogen and carbon monoxide;
(S4) introducing the first stream separated in (S3) to a second fluidized bed reactor and converting the first stream into carbon monoxide through a reverse Boudouard reaction;
(S5) mixing the second stream and the carbon monoxide converted in (S4) to prepare a third mixed gas;
(S6) producing a syngas from the third mixed gas through a water gas shift reaction; and
(S7) producing a hydrocarbon from the syngas through a catalytic reaction.

2. The method for preparing a hydrocarbon of claim 1, wherein the first mixed gas further includes one or more selected from the group consisting of landfill gas, shale gas, refinery exhaust gas, and biogas.

3. The method for preparing a hydrocarbon of claim 1, wherein the first stream includes 50 vol% or more of carbon dioxide.

4. The method for preparing a hydrocarbon of claim 1, wherein the pyrolysis gas has a C/O ratio of 0.1 or more.

5. The method for preparing a hydrocarbon of claim 1, wherein (S2) is performed under a composite catalyst in which an active metal is supported on a support.

6. The method for preparing a hydrocarbon of claim 5, wherein the active metal includes one or more selected from the group consisting of nickel, vanadium, iron, platinum, palladium, and ruthenium.

7. The method for preparing a hydrocarbon of claim 6, wherein the support includes one or more selected from the group consisting of silica, alumina, silica-alumina, carbon, zirconia, titania, zeolite, SAPO, and ALPO.

8. The method for preparing a hydrocarbon of claim 1, wherein (S2) is performed at a temperature of 700°C to 1000°C.

9. The method for preparing a hydrocarbon of claim 1, wherein (S4) is performed at a temperature of 600°C to 1000°C and a pressure of 50 to 300 KPa.

10. The method for preparing a hydrocarbon of claim 1, wherein the syngas includes hydrogen and carbon monoxide, and a ratio between the hydrogen and the carbon monoxide satisfies 1.8:1 to 2.2:1.

11. The method for preparing a hydrocarbon of claim 1, wherein the organic waste in (S1) is one or more selected from the group consisting of waste plastic, solid waste, biomass, waste oil, waste tire, and standard plastic garbage bags.

12. The method for preparing a hydrocarbon of claim 1, further comprising purifying the first mixed gas of (S1), before (S2).

13. An apparatus for preparing a hydrocarbon comprising:
a pyrolysis reactor which heat treats an organic waste to produce a first mixed gas;
a first fluidized bed reactor which dry reforms the first mixed gas under a catalyst to produce a second mixed gas;
a carbon dioxide separation unit which separates the second mixed gas into a first stream including carbon dioxide and a second stream including hydrogen and carbon monoxide;
a second fluidized bed reactor which converts the first stream into carbon monoxide through a reverse Boudouard reaction;
a gas mixing unit which mixes the second stream and carbon monoxide converted in the second fluidized bed reactor to prepare a third mixed gas;
a syngas production unit which converts the third mixed gas into a syngas through a water gas shift reaction; and
a hydrocarbon conversion unit which converts the syngas into a hydrocarbon through a catalytic reaction.

14. The apparatus for preparing a hydrocarbon of claim 13, further comprising:
a cyclone connected between the first fluidized bed reactor and the carbon dioxide separation unit;
a catalyst supply line which connects the cyclone and the second fluidized bed reactor; and
a catalyst recirculation line which connects the second fluidized bed reactor and the first fluidized bed reactor,
wherein the cyclone separates the second mixed gas and the catalyst discharged from the fluidized bed reforming reactor, supplies the second mixed gas to the carbon dioxide separation unit, and supplies the catalyst to the second fluidized bed reactor.

15. The apparatus for preparing a syngas of claim 13, further comprising a purification unit between the heat treatment reactor and the first fluidized bed reactor.
